# EUROPEAN PATENT SPECIFICATION

(11) **EP 4 006 007 B1**
(45) Date of publication and mention of the grant of the patent: **14.10.2026**
(21) Application number: 21849681.8
(22) Date of filing: 26.07.2021
(51) Int. Cl.: B01J 31/20, C07C 45/50, B01J 31/22, B01J 31/18, B01J 31/02, B01J 31/24, C07C 47/02

(54) **HYDROFORMYLATION METHOD**
HYDROFORMYLIERUNGSVERFAHREN
PROCÉDÉ D'HYDROFORMYLATION

(30) Priority: 30.07.2020 KR 20200095240
(43) Date of publication of application: 01.06.2022
(73) Proprietor: LG Chem, Ltd., Seoul 07336 (KR)
(72) Inventor: KWON, Yonghyun, Daejeon 34122 (KR); NAM, Hyunseok, Daejeon 34122 (KR); KIM, Jihoon, Daejeon 34122 (KR); CHO, Jangkeun, Daejeon 34122 (KR); CHOI, Daeheung, Daejeon 34122 (KR)
(74) Representative: Goddar, Heinz J.
(86) International application number: PCT/KR2021/009635
(87) International publication number: WO 2022/025562

(56) References cited:
- EP-A1- 0 028 892
- EP-B1- 0 071 281
- CN-A- 109 453 816
- KR-A- 19980 701 462
- KR-A- 19980 701 462
- KR-A- 20020 046 892
- KR-A- 20090 092 281
- US-A- 4 528 403
- US-A- 4 711 968
- BEHR ARNO ET AL: "Selective hydroformylation-hydrogenation tandem reaction of isoprene to 3-methylpentanal", DALTON TRANSACTIONS, vol. 40, no. 44, 1 January 2011 (2011-01-01), Cambridge, pages 11742, XP055972927, ISSN: 1477-9226, DOI: 10.1039/c1dt11292a

## Description

### [Technical Field]

The present application relates to a method for hydroformylation.

### [Background Art]

A hydroformylation reaction of reacting various olefins with carbon monoxide (CO) and hydrogen (H₂) commonly called as a synthetic gas under the presence of a homogeneous organometal catalyst and a ligand to produce a linear (normal) and branched (iso) aldehyde having the number of carbon atoms increased by one was first discovered by Otto Roelen of Germany in 1938.

The hydroformylation reaction, generally known as an oxo reaction, is an industrially very important reaction in a homogeneous catalyst reaction, and various aldehydes comprising alcohol derivatives are produced through the oxo process and consumed worldwide.

Various aldehydes synthesized through the oxo reaction are sometimes oxidized or hydrogenated after a condensation reaction such as an aldol, and modified to various acids and alcohols comprising a long alkyl group. Particularly, a hydrogenated alcohol of an aldehyde produced by such an oxo reaction is referred to as an oxo-alcohol, and the oxo-alcohol is widely used industrially as solvents, additives, raw materials of various plasticizers, synthetic lubricants and the like.

Catalysts currently used in the oxo process are mainly cobalt (Co) and rhodium (Rh) series, and normal/iso selectivity (ratio of linear (normal) to branched (iso) isomers) of a produced aldehyde varies depending on the types of ligand used and operating conditions. Currently, 70% or more of oxo plants around the world are adopting a low pressure oxo process using a rhodium-based catalyst.

As a central metal of the oxo catalyst, iridium (Ir), ruthenium (Ru), osmium (Os), platinum (Pt), palladium (Pd), iron (Fe), nickel (Ni) and the like may be used in addition to cobalt (Co) and rhodium (Rh). However, each of the metals are known to exhibit catalytic activity in the order of Rh>>Co>Ir, Ru>Os>Pt>Pd>Fe>Ni and the like, and accordingly, most processes and studies are focused on rhodium and cobalt.

As the ligand, phosphine (PR₃, R is C₆H₅, or n-C₄H₉), phosphine oxide (O=P(C₆H₅)₃), phosphide, amine, amide, isonitrile and the like may be used.

The hydroformylation reaction may be conducted continuously, semi-continuously or by the batch, and a typical hydroformylation reaction process is a gas or liquid recirculation system. In the hydroformylation reaction, it is important to increase reaction efficiency by allowing starting materials formed in liquid and gas phases to smoothly contact with each other. For this purpose, a continuous stirred tank reactor (CSTR) stirring components in liquid and gas phases to evenly contact with each other in the reactor has been mainly used in the art.

An existing hydroformylation process has two limits as follows.

The first disadvantage of an existing hydroformylation process is high unit costs of raw materials. Propylene, a raw material of an octanol process that is a typical hydroformylation process, is in high demand as a raw material of other polymers such as polypropylene, and the price continues to rise. The second disadvantage of an existing hydroformylation process is that concentrations of reaction materials introduced to a batch-type reaction are low and a concentration of a solvent is high. A solvent used in a hydroformylation process performs an important role of helping reaction materials in liquid and gas phases be in contact with a catalyst. In most literatures dealing with a hydroformylation process use a process condition of introducing small amounts of reaction materials and introducing a very large amount of solvent to obtain high activity. However, such a process condition has a problem of increasing raw material costs and causing a solvent separating cost in an economical aspect. Accordingly, studies for resolving the problems of an existing hydroformylation process described above have been required in the art.

EP 0 028 892 A1 relates to heteronuclear-bridge rhodium clusters useful as catalysts in the hydroformylation of olefins.

A. Behr et al. ("Selective hydroformylation-hydrogenation tandem reaction of isoprene to 3-methylpentanal", DALTON TRANSACTIONS, vol. 40, no. 44, 1 January 2011 (2011-01-01), page 11742, XP055972927, Cambridge ISSN: 1477-9226, DOI: 10.1039/c1dt11292a) discloses the hydroformylation of isoprene catalyzed by a rhodium complex with the chelate ligand bis(diphenylphosphino)ethane.in a selective auto-tandem reaction consisting of a hydroformylation and a hydrogenation step.

### [Disclosure]

### [Technical Problem]

The present application is directed to providing a method for hydroformylation.

### [Technical Solution]

One embodiment of the present application provides a method for hydroformylation, the method comprising hydroformylation of preparing an aldehyde by reacting a raw-C5 feed with a synthetic gas under the presence of a catalyst composition for a hydroformylation reaction,
wherein the catalyst composition for a hydroformylation reaction comprises a phosphorous-based ligand, a transition metal compound represented by the following Chemical Formula 1 or cobalt carbonyl [Co₂(CO)₈] and a solvent,
the solvent comprises one or more types of tetraethylene glycol dimethyl ether (TEGDME), 1,2,4-trimethylbenzene and cumene, and
the raw-C5 feed is a product of a naphtha cracking center (NCC) process, the raw-C5 feed comprises a mixture of terminal monoene, internal monoene and diene, wherein a content of the diene is 30% by weight or greater based on a total weight of the mixture of terminal monoene, internal monoene and diene; and the raw-C5 feed has a total weight ratio of less than 1,000 and the solvent has a total weight ratio of 500 or greater, based on a total weight of the transition metal compound:

   [Chemical Formula 1] M(L1)x(L2)y(L3)z
in Chemical Formula 1,
M is rhodium (Rh), cobalt (Co), iridium (Ir), ruthenium (Ru), iron (Fe), nickel (Ni), palladium (Pd), platinum (Pt) or osmium (Os),
L1, L2 and L3 are the same as or different from each other, and each independently hydrogen, carbonyl (CO), cyclooctadiene, norbornene, chlorine, triphenylphosphine (TPP) or acetylacetonato (AcAc), and
x, y and z are each independently an integer of 0 to 5, and x, y and z are not 0 at the same time.

### [Advantageous Effects]

According to one embodiment of the present application, a raw-C5 feed, a product of a naphtha cracking center (NCC) process, can be directly used in a hydroformylation reaction process. Accordingly, one embodiment of the present application is effective in saving operating costs and investment costs compared to existing technologies in the art.

In addition, according to one embodiment of the present application, an olefin conversion ratio in the raw-C5 feed can be enhanced by comprising one or more types of tetraethylene glycol dimethyl ether (TEGDME), 1,2,4-trimethylbenzene and cumene as a solvent, and by using a specific weight ratio of a transition metal compound, the solvent and the raw-C5 feed during the step of hydroformylation.

### [Mode for Disclosure]

Hereinafter, the present specification will be described in more detail.

In the present specification, a description of one member being placed "on" another member comprises not only a case of the one member being in contact with the another member but a case of still another member being present between the two members.

In the present specification, a description of a certain part "comprising" certain constituents means capable of further comprising other constituents, and does not exclude other constituents unless particularly stated on the contrary.

As described above, studies capable of resolving problems of an existing hydroformylation process described above have been required in the art.

Aldehydes used for making various alcohol and amine materials are generally synthesized through a hydroformylation process of olefins, and development of an economical hydroformylation process is very important for commercialization of aldehyde production. Raw-C5, one of products of a naphtha cracking center (NCC) process, is traded at a low price of a naphtha price level and has a high olefin content of 40% by weight to 50% by weight, and therefore, is a material having a high economic effect when converted to an aldehyde through a hydroformylation process. However, when several types of olefins with different reaction characteristics (internal monoene, terminal monoene, diene and the like) are mixed as in raw-C5, it is difficult to effectively produce an aldehyde under a single catalyst system, and an analysis on reaction results is difficult as well, and accordingly, there have been no related studies.

In view of the above, the present application is directed to providing a method for hydroformylation capable of saving operating costs and investment costs compared to existing technologies in the art and enhancing a conversion ratio of olefins by minimizing an amount of introduced solvent while using a raw-C5 feed.

A method for hydroformylation according to one embodiment of the present application comprises a step of hydroformylation of preparing an aldehyde by reacting a raw-C5 feed with a synthetic gas under the presence of a catalyst composition for a hydroformylation reaction, wherein the catalyst composition for a hydroformylation reaction comprises a phosphorous-based ligand, a transition metal compound represented by Chemical Formula 1 or cobalt carbonyl [Co₂(CO)₈] and a solvent, the solvent comprises one or more types of tetraethylene glycol dimethyl ether (TEGDME), 1,2,4-trimethylbenzene and cumene, and the raw-C5 feed is a product of a naphtha cracking center (NCC) process, the raw-C5 feed comprises a mixture of terminal monoene, internal monoene and diene, wherein a content of the diene is 30% by weight or greater based on a total weight of the mixture of terminal monoene, intemal monoene and diene; and the raw-C5 feed has a total weight ratio of less than 1,000 and the solvent has a total weight ratio of 500 or greater based on a total weight of the transition metal compound.

In one embodiment of the present application, specific components of the raw-C5 feed are shown in the following Table 1.

**[Table 1]**

| Components of Raw-C5 Feed | | | Content (wt%) |
|---|---|---|---|
| C5 Hydrocarbon | C5 Paraffin | | 36 to 42 |
| | C5 Olefin | C5 Terminal Monoene | 10 to 16 |
| | | C5 Internal Monoene | 2 to 8 |
| | | C5 Diene | 23 to 29 |
| Other Components (C4, C6, C7, C8, C9 Hydrocarbon and the like) | | | 5 to 29 |
| Total | | | 100 |

In one embodiment of the present application, the C5 terminal monoene may comprise 1-pentene, 2-methyl-1-butene, 3-methyl-1-butene, cyclopentene and the like. In addition, the C5 intemal monoene may comprise 2-pentene, 2-methyl-2-butene and the like. In addition, the C5 diene may comprise isoprene, 1,3-pentadiene, 1,4-pentadiene, 2-methyl-1-buten-3-yne and the like.

In one embodiment of the present application, a content of the diene may be from 40% by weight to 80% by weight, or from 45% by weight to 75% by weight based on a total weight of the mixture of terminal monoene, internal monoene and diene. Having the diene content of less than 30% by weight based on a total weight of the mixture of terminal monoene, internal monoene and diene is not preferred since the content of the internal monoene having relatively low reactivity increases resulting in a decrease in the olefin conversion ratio in the raw-C5 feed.

In one embodiment of the present application, the phosphorous-based ligand may comprise one or more types of 1,2-bis(diphenylphosphino)ethane, 4,5-bis(diphenylphosphino), triphenylphosphine and 6,6'-[(3,3'-di-tert-butyl-5,5'-dimethoxy-1,1'-biphenyl-2,2'-diyl)bis(oxy)]bis(dibenzo[d,f][1,3,2]dioxaphosphephine).

In one embodiment of the present application, the transition metal compound may comprise one or more types of acetylacetonatodicarbonyl rhodium [Rh(AcAc)(CO)₂], acetylacetonatocarbonyl triphenylphosphine rhodium [Rh(AcAc)(CO)(TPP)], hydridocarbonyl tri(triphenylphosphine)rhodium [HRh(CO)(TPP)₃], acetylacetonatodicarbonyl iridium [Ir(AcAc)(CO)₂], hydridocarbonyl tri(triphenylphosphine)iridium [HIr(CO)(TPP)₃] and chloro(1,5-cyclooctadiene)rhodium [Rh(COD)Cl₂].

According to the present invention, the solvent comprises one or more types of tetraethylene glycol dimethyl ether (TEGDME), 1,2,4-trimethylbenzene and cumene. Particularly, the TEGDME has a boiling point of 257.0°C and has a large boiling point difference of 117°C to 137°C from a C6 aldehyde (boiling point: 120°C to 140°C) produced after the step of hydroformylation. Accordingly, the solvent is readily separated through a distillation process after the step of hydroformylation, and therefore, using the TEGDME is more preferred.

According to the present invention, the raw-C5 feed has a total weight ratio of less than 1,000 and the solvent has a total weight ratio of 500 or greater based on a total weight of the transition metal compound. In addition, in one embodiment of the present application, the raw-C5 feed preferably has a total weight ratio of greater than or equal to 50 and less than 1,000 and the solvent preferably has a total weight ratio of 500 to 1,200 based on a total weight of the transition metal compound. In addition, the raw-C5 feed more preferably has a total weight ratio of greater than or equal to 100 and less than or equal to 900 and the solvent more preferably has a total weight ratio of 500 to 1,000 based on a total weight of the transition metal compound. Economic feasibility of the hydroformylation process increases as the raw-C5 feed has a large weight ratio and the solvent has a small weight ratio with respect to a weight of the transition metal compound. However, when the solvent has a total weight ratio of less than 500 based on a total weight of the transition metal compound, the transition metal compound is not sufficiently dissolved failing to function as a catalyst, and as a result, the olefin conversion ratio may be low. In addition, when the solvent has a total weight ratio of greater than 1,000 based on a total weight of the transition metal compound, the transition metal compound is sufficiently dissolved and catalytic activity may be obtained, however, the raw-C5 feed having a very high total weight ratio of 1,000 or greater is not preferred since the relative concentration of the catalyst is low with a limited amount resulting in insufficient olefin conversion.

In one embodiment of the present application, the step of hydroformylation may be conducted at a reaction temperature of 90°C or higher, may be conducted at a reaction temperature of 90°C to 120°C, and may be conducted at a reaction temperature of 90°C to 110°C. In addition, in one embodiment of the present application, the step of hydroformylation may be conducted at a reaction pressure of 15 bar or greater, may be conducted at a reaction pressure of 20 bar to 60 bar, and may be conducted at a reaction pressure of 20 bar to 50 bar.

When the step of hydroformylation has a reaction temperature of lower than 90°C, energy required for the reaction is not sufficiently supplied, and conversion of olefin molecules having low reactivity such as internal monoene or diene may slowly occur or hardly occur. In addition, even with terminal monoene, the reaction time required for conversion is too long, and production efficiency may decrease. In addition, the step of hydroformylation having a reaction temperature of higher than 120°C is not preferred since the catalyst may not function normally due to thermal composition of the ligand molecule.

When the step of hydroformylation has a reaction pressure of 15 bar or greater, the equivalent of the synthetic gas is sufficiently high with respect to the olefin, which allows the hydroformylation reaction equilibrium to create a condition advantageous for a forward reaction. In addition, C5 olefin molecules having a low boiling point exist in a liquid phase even at high temperatures, which may be advantageous for contact with the catalyst in the reaction solution. In addition, when the step of hydroformylation has a reaction pressure of less than 15 bar, conversion of olefin molecules having low reactivity such as internal monoene or diene may slowly occur or hardly occur. In addition, the step of hydroformylation having a reaction pressure of greater than 60 bar is not preferred since, although it does not cause problems in the reaction, investment costs such as strengthening a reactor for a high pressure process may additionally incur, and risks of a high pressure process may increase.

In the step of hydroformylation in one embodiment of the present application, a molar ratio of the raw-C5 feed:the synthetic gas may be from 95:5 to 5:95.

In one embodiment of the present application, the synthetic gas comprises carbon monoxide and hydrogen, and a molar ratio of the carbon monoxide:the hydrogen may be from 5:95 to 70:30, and from 40:60 to 60:40. When satisfying the molar ratio of the carbon monoxide:the hydrogen, the olefin conversion ratio in the raw-C5 feed may be further enhanced.

Hereinafter, the present application will be described in detail with reference to examples in order to specifically describe the present application. Examples of the present application are provided in order to more fully describe the present application to those having average knowledge in the art.

### <Example>

### <Example 1>

A C5 stream solution (10 g) collected from the Daesan NCC plant of LG Chem., a catalyst precursor, a ligand and a solvent were mixed in a fixed ratio, and introduced to a pressure reactor (capacity: 500 mL). 0.1 g of Rhacac(CO)₂ was used as the catalyst precursor, and 2 g of 1,2-bis(diphenylphosphino)ethane (DPPE) was used as the ligand. As the solvent, 100 g of tetraethylene glycol dimethyl ether (TEGDME) was used. Components of the C5 stream solution are as shown in Table 1 described above.

Inside the reactor was purged with nitrogen while stirring the reaction solution at 1,000 rpm. When the purging was completed, the temperature inside the reactor was raised to 100°C. After reaching the reaction temperature, a synthetic gas (CO/H₂=1/1) was introduced into the reactor at 40 bar to initiate a hydroformylation reaction. After keeping the result for 12 hours under the hydroformylation reaction condition, the reactor was cooled to room temperature, and the gas inside the reactor was removed to finish the reaction.

### <Examples 2 to 6 and Comparative Examples 1 to 7>

Reactions were performed in the same manner as in Example 1 except for using the solvents, the amounts of the introduced solvents and the amounts of the introduced C5 stream solutions as described in the following Table 2.

**[Table 2]**

| | Solvent | Amount of Introduced C5 Stream | Amount of Introduced Solvent | A | B |
|---|---|---|---|---|---|
| Example 1 | TEGDME | 10 | 100 | 100 | 1,000 |
| Example 2 | Pseudocumene | 10 | 100 | 100 | 1,000 |
| Example 3 | Cumene | 10 | 100 | 100 | 1,000 |
| Example 4 | TEGDME | 75 | 50 | 750 | 500 |
| Example 5 | TEGDME | 50 | 50 | 500 | 500 |
| Example 6 | TEGDME | 90 | 50 | 900 | 500 |
| Comparative Example 1 | Benzene | 10 | 100 | 100 | 1,000 |
| Comparative Example 2 | TEXANOL | 10 | 100 | 100 | 1,000 |
| Comparative Example 3 | TEGDME | 100 | 100 | 1,000 | 1,000 |
| Comparative Example 4 | TEGDME | 100 | 50 | 1,000 | 500 |
| Comparative Example 5 | TEGDME | 50 | 25 | 500 | 250 |
| Comparative Example 6 | TEGDME | 50 | 0 | 500 | 0 |
| Comparative Example 7 | TEGDME | 50 | 40 | 500 | 400 |

| | | | | | |
|---|---|---|---|---|---|
| A: total weight ratio of C5 stream with respect to total weight of transition metal compound B: total weight ratio of solvent with respect to total weight of transition metal compound TEGDME: tetraethylene glycol dimethyl ether Pseudocumene: 1,2,4-trimethylbenzene Cumene: isopropylbenzene TEXANOL: 2,2,4-trimethyl-1,3-pentanediol monoisobutyrate | | | | | |

### <Experimental Example>

After completing the reactions according to the examples and the comparative examples, each of the reaction solutions was collected, and olefin conversion ratio and aldehyde selectivity were calculated through gas chromatography (GC). The results are shown in the following Table 3.

The olefin conversion ratio was calculated through the total consumption ratio of C5 olefins present in the raw-C5 feed before and after the reaction. In addition, the aldehyde selectivity was calculated as the amount of total C6 aldehydes produced after the reaction with respect to the total consumption of C5 olefins present in the raw-C5 feed.

Olefin conversion ratio (%)=[(number of moles of reacted C5 olefins)/(number of moles of C5 olefins present in supplied raw-C5 feed)]x100

Aldehyde selectivity (%)=[(number of moles of produced C6 aldehydes)/(number of moles of reacted C5 olefins)]x100

### <Condition of GC Analysis>

1) Column: HP-1 (L: 30 m, ID: 0.32 mm, film: 1.05 m)
2) Injection volume: 1 µl
3) Inlet temp.: 250°C, pressure: 6.92 psi, total flow: 64.2 ml/min, split flow: 60 ml/min, split ratio: 50:1
4) Column flow: 1.2 ml/min
5) Oven temp.: 50°C/3 min-10°C/min-280°C/41 min (total 67 mins)
6) Detector temp.: 300°C, H2: 35 ml/min, air: 300 ml/min, He: 20 ml/min
7) GC model: Agilent 6890

**[Table 3]**

| | Olefin Conversion Ratio (%) | Aldehyde Selectivity (%) |
|---|---|---|
| Example 1 | 78.1 | 99.1 |
| Example 2 | 79.5 | 64.2 |
| Example 3 | 75.7 | 86.6 |
| Example 4 | 75.0 | 79.8 |
| Example 5 | 73.9 | 72.0 |
| Example 6 | 66.8 | 70.1 |
| Comparative Example 1 | 32.7 | 40.0 |
| Comparative Example 2 | 10.4 | 17.3 |
| Comparative Example 3 | 10.6 | 93.5 |
| Comparative Example 4 | 8.1 | 36.4 |
| Comparative Example 5 | 20.0 | 37.9 |
| Comparative Example 6 | 13.3 | 51.8 |
| Comparative Example 7 | 64.3 | 58.1 |

As in Examples 1 to 6, it was identified that superior olefin conversion ratio and aldehyde selectivity were obtained when using TEGDME, 1,2,4-trimethylbenzene or cumene as the solvent compared to when using benzene or TEXANOL as the solvent as in Comparative Examples 1 and 2.

In addition, as in Comparative Examples 3 to 7, it was identified that the hydroformylation reaction did not proceed well when the total weight ratio of the raw-C5 feed and the total weight ratio of the solvent were not satisfied based on the total weight of the transition metal compound of the present application.

Particularly, as in the result of Comparative Example 4, it was identified that olefin conversion ratio and aldehyde selectivity rapidly decreased when the total weight ratio of the C5 stream with respect to the total weight of the transition metal compound was 1,000 or greater. In addition, as in the result of Comparative Example 3, it was identified that, even when the total weight ratio of the solvent with respect to the total weight of the transition metal compound was 1,000 or greater, reaction activity was not secured when the total weight ratio of the C5 stream with respect to the total weight of the transition metal compound was 1,000 or greater. In addition, as in the results of Comparative Examples 5 to 7, it was identified that reaction activity was not secured when the total weight ratio of the solvent with respect to the total weight of the transition metal compound was less than 500.

Accordingly, the raw-C5 feed, a product of a naphtha cracking center (NCC) process, may be directly used in a hydroformylation reaction process according to one embodiment of the present application. As a result, one embodiment of the present application is effective in saving operating costs and investment costs compared to existing technologies in the art.

In addition, according to one embodiment of the present application, an olefin conversion ratio in the raw-C5 feed may be enhanced by comprising one or more types of tetraethylene glycol dimethyl ether (TEGDME), 1,2,4-trimethylbenzene and cumene as the solvent, and by using a specific weight ratio of the transition metal compound, the solvent and the raw-C5 feed during the step of hydroformylation.

## Claims

1. A method for hydroformylation, the method comprising hydroformylation of preparing an aldehyde by reacting a raw-C5 feed with a synthetic gas under the presence of a catalyst composition for a hydroformylation reaction,
wherein the catalyst composition for the hydroformylation reaction comprises a phosphorous-based ligand, a transition metal compound represented by the following Chemical Formula 1 or cobalt carbonyl [Co₂(CO)₈] and a solvent;
the solvent comprises one or more types of tetraethylene glycol dimethyl ether (TEGDME), 1,2,4-trimethylbenzene and cumene; and
the raw-C5 feed is a product of a naphtha cracking center (NCC) process, the raw-C5 feed comprises a mixture of terminal monoene, internal monoene and diene, wherein a content of the diene is 30% by weight or greater based on a total weight of the mixture of terminal monoene, internal monoene and diene; and the raw-C5 feed has a total weight ratio of less than 1,000 and the solvent has a total weight ratio of 500 or greater, based on a total weight of the transition metal compound:
[Chemical Formula 1] M(L1)x(L2)y(L3)z
in Chemical Formula 1,
M is rhodium (Rh), cobalt (Co), iridium (Ir), ruthenium (Ru), iron (Fe), nickel (Ni), palladium (Pd), platinum (Pt) or osmium (Os);
L1, L2 and L3 are the same as or different from each other, and each independently hydrogen, carbonyl (CO), cyclooctadiene, norbornene, chlorine, triphenylphosphine (TPP) or acetylacetonato (AcAc); and
x, y and z are each independently an integer of 0 to 5, and x, y and z are not 0 at the same time.

2. The method for hydroformylation of Claim 1, wherein the phosphorous-based ligand comprises one or more types of 1,2-bis(diphenylphosphino)ethane, 4,5-bis(diphenylphosphino), triphenylphosphine and 6,6'-[(3,3'-di-tert-butyl-5,5'-dimethoxy-1,1'-biphenyl-2,2'-diyl)bis(oxy)]bis(dibenzo[d,f][1,3,2]dioxaphosphephine).

3. The method for hydroformylation of Claim 1, wherein the transition metal compound comprises one or more types of acetylacetonatodicarbonyl rhodium [Rh(AcAc)(CO)₂], acetylacetonatocarbonyl triphenylphosphine rhodium [Rh(AcAc)(CO)(TPP)], hydridocarbonyl tri(triphenylphosphine)rhodium [HRh(CO)(TPP)₃], acetylacetonatodicarbonyl iridium [Ir(AcAc)(CO)₂], hydridocarbonyl tri(triphenylphosphine)iridium [HIr(CO)(TPP)₃] and chloro(1,5-cyclooctadiene)rhodium [Rh(COD)Cl₂].

4. The method for hydroformylation of Claim 1, wherein the hydroformylation is conducted at a reaction temperature of 90°C or higher and a reaction pressure of 15 bar or higher.

5. The method for hydroformylation of Claim 1, wherein a molar ratio of the raw-C5 feed:the synthetic gas is from 95:5 to 5:95.

6. The method for hydroformylation of Claim 1, wherein the synthetic gas comprises carbon monoxide and hydrogen, and a molar ratio of the carbon monoxide:the hydrogen is from 5:95 to 70:30.

## Patentansprüche

1. Verfahren zur Hydroformylierung, wobei das Verfahren die Hydroformylierung zur Herstellung eines Aldehyds durch Umsetzen eines rohen C5-Einsatzmaterials mit einem Synthesegas in Gegenwart einer Katalysatorzusammensetzung für eine Hydroformylierungsreaktion umfasst,
wobei die Katalysatorzusammensetzung für die Hydroformylierungsreaktion einen Liganden auf Basis von Phosphor, eine Übergangsmetallverbindung der folgenden chemischen Formel 1 oder Cobaltcarbonyl [Co₂(CO)₈] und ein Lösungsmittel umfasst;
das Lösungsmittel eine oder mehrere Arten von Tetraethylenglycoldimethylether (TEGDME), 1,2,4-Trimethylbenzol und Cumol umfasst; und
das rohe C5-Einsatzmaterial ein Produkt eines Naphthacrackcenter(NCC)-Verfahrens ist, das rohe C5-Einsatzmaterial eine Mischung aus terminalem Monoen, internem Monoen und Dien umfasst, wobei ein Gehalt des Diens 30 Gew.-% oder mehr, bezogen auf ein Gesamtgewicht der Mischung aus terminalem Monoen, internem Monoen und Dien, beträgt; und das rohe C5-Einsatzmaterial ein Gesamtgewichtsverhältnis von weniger als 1.000 aufweist und das Lösungsmittel ein Gesamtgewichtsverhältnis von 500 oder mehr aufweist, bezogen auf ein Gesamtgewicht der Übergangsmetallverbindung:
[Chemische Formel 1] M(L1)x(L2)y(L3)z
wobei in der chemischen Formel 1
M Rhodium (Rh), Cobalt (Co), Iridium (Ir), Ruthenium (Ru), Eisen (Fe), Nickel (Ni), Palladium (Pd), Platin (Pt) oder Osmium (Os) ist;
L1, L2 und L3 gleich oder voneinander verschieden sind und jeweils unabhängig Wasserstoff, Carbonyl (CO), Cyclooctadien, Norbornen, Chlor, Triphenylphosphin (TPP) oder Acetylacetonat (AcAc) sind; und
x, y und z jeweils unabhängig eine ganze Zahl von 0 bis 5 sind und x, y und z nicht gleichzeitig 0 sind.

2. Verfahren zur Hydroformylierung nach Anspruch 1, bei dem der Ligand auf Basis von Phosphor eine oder mehrere Arten von 1,2-Bis(diphenylphosphino)ethan, 4,5-Bis(diphenylphosphino), Triphenylphosphin und 6,6'-[(3,3'-Di-tert-butyl-5,5'-dimethoxy-1,1'-biphenyl-2,2'-diyl)bis(oxy)]bis(dibenzo[d,f][1,3,2]dioxaphosphephin) umfasst.

3. Verfahren zur Hydroformylierung nach Anspruch 1, bei dem die Übergangsmetallverbindung eine oder mehrere Arten von Acetylacetonatodicarbonylrhodium [Rh(AcAc)(CO)₂], Acetylacetonatocarbonyltriphenylphosphinrhodium [Rh(AcAc)(CO)(TPP)], Hydridocarbonyltri(triphenylphosphin)rhodium [HRh(CO)(TPP)₃], Acetylacetonatodicarbonyliridium [Ir(AcAc)(CO)₂], Hydridocarbonyltri(triphenylphosphin)iridium [HIr(CO)(TPP)₃] und Chlor(1,5-cyclooctadien)rhodium [Rh(COD)Cl₂] umfasst.

4. Verfahren zur Hydroformylierung nach Anspruch 1, bei dem die Hydroformylierung bei einer Reaktionstemperatur von 90°C oder höher und einem Reaktionsdruck von 15 bar oder höher durchgeführt wird.

5. Verfahren zur Hydroformylierung nach Anspruch 1, bei dem das Molverhältnis des rohen C5-Einsatzmaterials:Synthesegas 95:5 bis 5:95 beträgt.

6. Verfahren zur Hydroformylierung nach Anspruch 1, bei dem das Synthesegas Kohlenmonoxid und Wasserstoff umfasst und das Molverhältnis des Kohlenmonoxids zu Wasserstoff 5:95 bis 70:30 beträgt.

## Revendications

1. Procédé d'hydroformylation, le procédé comprenant une hydroformylation de préparation d'un aldéhyde en faisant réagir un flux de brut-C5 avec un gaz de synthèse en présence d'une composition catalytique pour une réaction d'hydroformylation,
dans lequel la composition catalytique pour la réaction d'hydroformylation comprend un ligand à base de phosphore, un composé de métal de transition représenté par la Formule 1 ci-dessous ou un cobaltcarbonyle [Co₂(CO)₈] et un solvant ;
le solvant comprend un ou plusieurs types d'éther diméthylique de tétraéthylèneglycol (TEGDME), 1,2,4-triméthylbenzène et cumène ; et
le flux de brut-C5 est un produit d'un processus de craquage catalytique de naphta (CCN), le flux de brut-C5 comprend un mélange de monoène terminal, monoène interne et diène, dans lequel une teneur du diène est égale ou supérieure à 30 % en poids sur la base d'un poids total du mélange de monoène terminal, monoène interne et diène ; et le flux de brut-C5 a un rapport de poids total inférieur à 1000 et le solvant a un rapport de poids total égal ou supérieur à 500, sur la base d'un poids total du composé de métal de transition :
[Formule chimique 1] M(L1)x(L2)y(L3)z
dans la Formule chimique 1,
M est un rhodium (Rh), cobalt (Co), iridium (Ir), ruthénium (Ru), fer (Fe), nickel (Ni), palladium (Pd), platine (Pt) ou osmium (Os) ;
L1, L2 et L3 sont identiques ou différents les uns des autres, et chacun indépendamment un hydrogène, carbonyle (CO), cyclooctadiène, norbornène, chlore, triphénylphosphine (TPP) ou acétylacétonate (AcAc) ; et
x, y et z sont chacun indépendamment un nombre entier de 0 à 5, et x, y et z ne sont pas simultanément 0.

2. Procédé d'hydroformylation selon la revendication 1, dans lequel le ligand à base de phosphore comprend un ou plusieurs types de 1,2-bis(diphénylphosphino)éthane, 4,5-bis(diphénylphosphino), triphénylphosphine et 6,6'-[(3,3'-di-tert-butyl-5,5'-diméthoxy-1,1'-biphényl-2,2'-diyl)bis(oxy)]bis(dibenzo[d,f][1,3,2]dioxaphosphephine).

3. Procédé d'hydroformylation selon la revendication 1, dans lequel le composé de métal de transition comprend un ou plusieurs types de rhodium acétylacétonate dicarbonyle [Rh(AcAc)(CO)₂], rhodium triphénylphosphine acétylacétonate carbonyle [Rh(AcAc)(CO)(TPP tristriphénylphosphine carbonyle hydrorhodium [HRh(CO)(TPP)₃], acétylacétonatodicarbonyle iridium [Ir(AcAc)(CO)₂], hydridocarbonyle tri(triphénylphosphine)iridium [HIr(CO)(TPP)₃] et chloro(1,5-cyclooctadiène)rhodium [Rh(COD)Cl₂].

4. Procédé d'hydroformylation selon la revendication 1, dans lequel l'hydroformylation est effectuée à une température de réaction égale ou supérieure à 90°C et une pression de réaction égale ou supérieure à 15 bar.

5. Procédé d'hydroformylation selon la revendication 1, dans lequel un rapport molaire de flux de brut-C5:gaz de synthèse est de 95:5 à 5:95.

6. Procédé d'hydroformylation selon la revendication 1, dans lequel le gaz de synthèse comprend un monoxyde de carbone et un hydrogène, et un rapport molaire du monoxyde de carbone:hydrogène est de 5:95 à 70:30.
